# EUROPEAN PATENT SPECIFICATION

(11) **EP 1 610 787 B1**
(45) Date of publication and mention of the grant of the patent: **23.01.2008**
(21) Application number: 04723608.8
(22) Date of filing: 26.03.2004
(51) Int. Cl.: A61K 31/46, A61P 11/00, A61K 31/44

(54) **SYNERGISTIC COMBINATION COMPRISING ROFLUMILAST AND AN ANTICHOLINERGIC AGENT SELECTED FROM TIOTROPIUM SALTS FOR THE TREATMENT OF RESPIRATORY DISEASES**
SYNERGISTISCHE KOMBINATION ENTHALTEND ROFLUMILAST UND EINEN ANTICHOLINERGISCHEN WIRKSTOFF AUSGEWÄHLT AUS TIOTROPIUMSALZEN FÜR DIE BEHANDLUNG VON ATEMWEGSERKRANKUNGEN
COMBINAISON SYNERGIQUE COMPRENANT LE ROFLUMILAST ET UNE SUBSTANCE ANTICHOLINERGIQUE SELECTIONEE PARMI LE GROUPE DE SELS DE TIOTROPIUM POUR LE TRAITEMENT DE MALADIES RESPIRATOIRES

(30) Priority: 28.03.2003 EP 03007104
(43) Date of publication of application: 04.01.2006
(73) Proprietor: Nycomed GmbH, 78467 Konstanz (DE)
(72) Inventor: BUNDSCHUH, Daniela, CH - 8272 Ermatingen (CH); WOLLIN, Stefan-Lutz, 88709 Meersburg (DE); WEIMAR, Christian, 78467 Konstanz (DE)
(74) Representative: Wild, Robert
(86) International application number: PCT/EP2004/050376
(87) International publication number: WO 2004/084896

(56) References cited:
- WO-A-02/069945
- WO-A-02/096423
- WO-A-02/096463
- WO-A-03/011274

## Description

### Field of application of the invention

The invention relates to the combination of certain known active compounds for therapeutic purposes. The substances used in the combination according to the invention are a known active compound from the PDE inhibitor class and active compounds from the anticholinergic agent class.

### Prior art

International patent applications WO02/069945 and WO03/011274 generally describe the combination of a compound from the class of PDE4 inhibitors with a compound from the class of anticholinergic agents for the treatment of respiratory tract disorders. International Patent application WO02/096463 describes an inhaled combination of a selective PDE4 inhibitor and an anticholinergic agent, with the proviso that the anticholinergic agent is not a tiotropium salt. International patent application WO02/096423 describes a combination of therapeutic agents useful in the treatment of obstructive airways and other inflammatory diseases comprising (I) a PDE4 inhibitor that is therapeutically effective in the treatment of said diseases when administered by inhalation; together with (II) an anticholinergic agent comprising a member selected from the group consisting of tiotropium and derivatives thereof that is therapeutically effective in the treatment of said diseases when administered by inhalation. In the US patent application No. US2002/0052312 a method for the treatment of chronic obstructive pulmonary disease is described comprising administering orally to a patient in need of such treatment a therapeutically effective amount of a muscarinic receptor antagonist in combination with a therapeutically effective amount of at least one other therapeutic agent selected from the group consisting of: β2-agonist, antitussive, corticosteroid, decongestant, histamine H1 antagonist, dopamine antagonist, leukotriene antagonist, 5-lipoxygenase inhibitor, phosphodiesterase IV inhibitor, VLA-4 antagonist, and theophylline.

### Summary of the invention

The invention relates to pharmaceutical compositions and use thereof for preventing or reducing the onset of symptoms of respiratory diseases, or treating or reducing the severity of respiratory diseases. In particular it relates to compositions and use thereof for treating respiratory diseases mediated by phosphodiesterase 4 (PDE4) by administering a PDE4 inhibitor together with another pharmaceutically active agent, which affects pulmonary function. In this connection, it is the object of the present invention to make available a certain respiratory tract therapeutic, which fulfills the following conditions:
- Pronounced antiinflammatory action
- Distinct bronchorelaxation and -dilatation
- Good bioavailability
- Minor side effects
- Good suitability for long-term therapy
- Favorable influence on bronchial hyperreactivity

It has now been found that the combined use of the PDE4 inhibitor roflumilast and an anticholinergic agent selected from tiotropium bromide and tiotropium bromide monohydrate outstandingly fulfills the abovementioned conditions, in particular in view of the fact that the combination of the compounds acts synergistically, i. e. exhibits a greater than additive effect.

The invention relates to a pharmaceutical composition suited for administration by inhalation comprising an effective amount of roflumilast, an effective amount of an anticholinergic agent selected from tiotropium bromide and tiotropium bromide monohydrate together with pharmaceutically acceptable excipients and/or carriers.

The invention furthermore relates to the use of a combination of roflumilast and an anticholinergic agent selected from tiotropium bromide and tiotropium bromide monohydrate for the preparation of a pharmaceutical composition for preventing or reducing the onset of symptoms of a respiratory disease, or treating or reducing the severity of a respiratory disease.

### Detailed description of the invention

The combination therapy which is the subject matter of this invention comprises administering roflumilast with an anticholinergic agent selected from tiotropium bromide and tiotropium bromide monohydrate to prevent the onset of a respiratory disease event or to treat an existing condition. The two compounds are administered intranasal (e.g. in form of a nasal spray) or by inhalation together in a single dosage form; or they are administered intranasal (e.g. in form of a nasal spray) or by inhalation in two different dosage forms. They may be administered at the same time. Or they may be administered both close in time or remotely, such as where one active compound is administered in the morning and the second active compound is administered in the evening.

The combination may be used prophylactic or after the onset of symptoms has occurred. In some instances the combination may be used to prevent the progression of a respiratory disease or to arrest the decline of a function such as lung function.

The invention thus relates to the combined use of roflumilast and an anticholinergic agent selected from tiotropium bromide and tiotropium bromide monohydrate in preventing the symptoms of, or treating a respiratory disease.

In the sense of the invention, the term "roflumilast" is understood to include the pharmaceutically acceptable salts and the N-oxide of ROFLUMILAST, which can likewise be used according to the invention.

ROFLUMILAST is the international nonproprietary name (INN) for 3-cyclopropylmethoxy-4-difluoromethoxy-N-(3,5-dichloropyrid-4-yl)benzamide [structure of formula (1.1)]. The preparation of 3-cyclopropylmethoxy-4-difluoromethoxy-N-(3,5-dichloropyrid-4-yl)benzamide, its pharmaceutically acceptable salts and its N-oxide [3-cyclopropylmethoxy-4-difluoromethoxy-N-(3,5-dichloro-1-xypyrid-4-yl)-benzamide; structure of formula (1.2)] as well as the use of these compounds as phosphodiesterase (PDE) 4 inhibitors is described in WO95/01338.

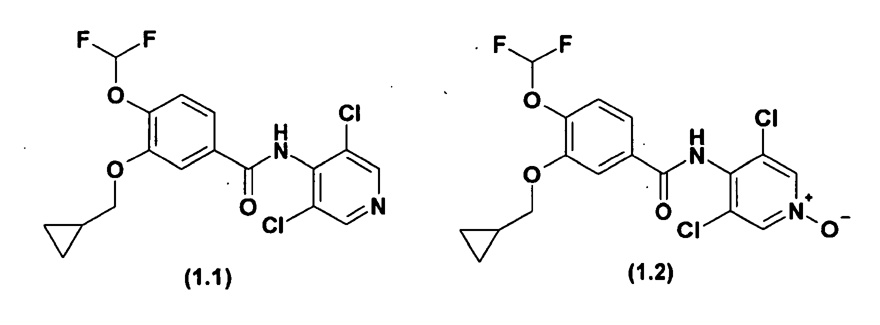

Suitable pharmaceutically acceptable salts of 3-cyclopropylmethoxy-4-difluoromethoxy-N-(3,5-dichloropyrid-4-yl)benzamide (ROFLUMILAST) are in particular water-soluble and water-insoluble acid addition salts with acids such as, for example, hydrochloric acid, hydrobromic acid, phosphoric acid, nitric acid, sulfuric acid, acetic acid, citric acid, D-gluconic acid, benzoic acid, 2-(4-hydroxybenzoyl)-benzoic acid, butyric acid, sulfosalicylic acid, maleic acid, lauric acid, malic acid, fumaric acid, succinic acid, oxalic acid, tartaric acid, embonic acid, stearic acid, toluenesulfonic acid, methanesulfonic acid or 1-hydroxy-2-naphthoic acid, the acids being employed in salt preparation -depending on whether it is a mono- or polybasic acid and depending on which salt is desired - in an equimolar quantitative ratio or one differing therefrom.

Anticholinergic agents suitable for use in the invention are tiotropium salts.

A tiotropium salt (see EP 418716) has the structure of formula (1.5): 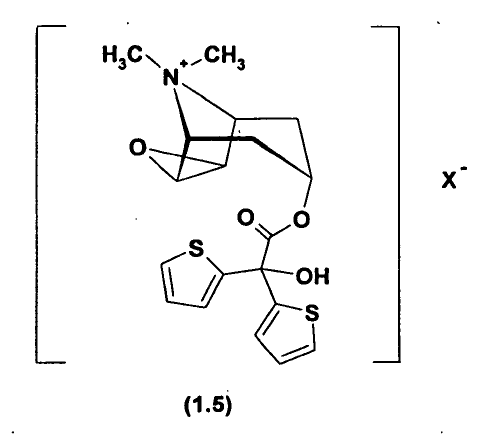 wherein X⁻ is a pharmaceutically acceptable anion.

The bromide salt is preferred.

Preferred combinations for use in the invention include:
- roflumilast and a tiotropium salt, particularly tiotropium bromide or tiotropium bromide monohydrate

It is understood that the active compounds and their pharmaceutically acceptable salts mentioned can also be present, for example, in the form of their pharmaceutically acceptable solvates, in particular in the form of their hydrates. Of particular importance in this connection is tiotropium bromide in form of its crystalline monohydrate as disclosed and described in detail in WO02/30928. The preparation of crystalline water-free tiotropium bromide is described in WO03/000265. An alternative process for the preparation of tiotropium bromide is described in WO02/051840.

Respiratory diseases which may be mentioned are in particular allergen- and inflammation-induced bronchial disorders (bronchitis, obstructive bronchitis, spastic bronchitis, allergic bronchitis, allergic asthma, bronchial asthma, COPD), which can be treated by the combination according to the invention also in the sense of a long-term therapy (if desired with appropriate adjustment of the dose of the individual components to the needs at the time, for example needs subject to seasonally related variations). The combination is particularly useful in the treatment of COPD.

"Combined use" or "combination" within the meaning of the present invention is to be understood as meaning that the individual components can be administered simultaneously (in the form of a combination medicament -fixed combination), or in succession (from separate pack units -free combination), close in time or remote in time, in any order whatever. As an example, one active compound could be taken in the morning and one later in the day. Or in another scenario, one active compound could be taken twice daily and the other once daily, either at the same time as one of the twice-a-day dosing occurred, or separately. Preferred is the once daily administration of the fixed combination. In case of administration in succession of the two active compounds it is preferred that the anticholinergic agent is administered first and roflumilast thereafter.

"Combined use" or "combination" within the meaning of the present invention is particularly to be understood as meaning that the two active compounds act together in a synergistic manner.

"Use" in accordance with the invention is to be understood to mean administration of the active compounds by inhalation (via mouth or via nose) or intranasal administration. As suitable administration forms for inhalation may be mentioned inhalation powders, propellant-containing aerosols and propellant-free inhalation solutions and/or suspensions. As suitable intranasal administration form may be mentioned, for example, a nasal spray or liquids for nasal administration (e. g. nose drops).

Thus, the invention contemplates, for example, either co-administering both active compounds in one delivery form such as an inhaler, which is putting both active compounds in the same inhaler, or alternatively putting the both active compounds in two different inhalers. Or, as a further alternative, administering one active compound intranasally, for example in form of a nasal spray, and the other active compound by inhalation.

The selective PDE4 inhibitors and the anticholinergic agents of the present invention may be conveniently delivered in the form of a dry powder inhaler or an aerosol spray presentation from a pressurized container, pump, spray, atomizer (preferably an atomizer using electrodynamics to produce a fine mist) or nebulizer, with or without the use of a suitable propellant, e. g. dichlorodifluoromethane, trichlorofluoromethane, dichlorotetrafluoroethane, a hydrofluoroalkane such as 1,1,2,2-tetrafluoroethane (HFA 134A [trade mark]) or, 1,1,1,2,3,3,3-heptafluoropropane (HFA 227EA [trade mark]), carbon dioxide, a further perfluorinated hydrocarbon such as Perflubon [trade mark] or other suitable gas. In the case of a pressurized aerosol, the dosage unit may be determined by providing a valve to deliver a metered dose. The pressurized container, pump, spray, or nebulizer may contain a solution or suspension of the selective PDE4 inhibitor and/or the anticholinergic agent, e. g. using a mixture of ethanol (optionally aqueous ethanol) or a suitable agent for dispersing, solubilizing or extending release and the propellant as the solvent, which may additionally contain a lubricant, e.g. sorbitan trioleate. Capsules, blisters and cartridges (made, for example, from gelatin or HMPC) for use in an inhaler or insufflator may be formulated to contain a powder mix of the selective PDE4 inhibitor and/or the anticholinergic agent of the invention, a suitable powder base, such as lactose or starch and a performance modifier such as I-leucine, mannitol or magnesium stearate.

Prior to use in a dry powder formulation for inhalation selective PDE4 inhibitors and the anticholinergic agents of the invention will be micronised to a size suitable for delivery by inhalation (typically considered as less than 5 microns). Micronisation could be achieved by a range of methods, for example spiral jet milling, fluid bed jet milling or use of supercritical fluid crystallization.

A suitable solution formulation for use in an atomizer using electrohydrodynamics to produce a finemist may contain from 1 µg to 10 mg of an anticholinergic agent of the invention and the actuation volume may vary from 1 to 100 µl. A typical formulation may comprise an anticholinergic agent of the invention, propylene glycol, sterile water, ethanol and sodium chloride.

Aerosol or dry powder formulations are preferably arranged so that each metered dose or "puff" contains from 1 to 4000 µg of an anticholinergic agent of the invention for delivery to the patient. The overall daily dose with an aerosol will be in the range from 1 µg to 20 mg which may be administered in a single dose or, alternatively, in divided doses throughout the day.

Typical formulations for intranasal administration include those mentioned above for inhalation and further include non-pressurized formulations in form of a solution or suspension in an inert vehicle such as water optionally in combination with conventional excipients such as buffers, anti-microbials, tonicity modifying agents and viscosity modifying agents, which may be administered by a nasal pump.

With respect to tiotropium bromide or tiotropium bromide monohydrate suitable tiotropium-containing powdery preparations for inhalative administration are disclosed in the international applications WO02/30389 and WO03/084509. In the international application WO02/098874 inhalation capsules (Inhalettes) containing the active agent tiotropium in the form of a powder preparation are disclosed. Propellant-free inhalation formulations of tiotropium bromide or tiotropium bromide monohydrate are disclosed in the international applications WO02/36104 and WO02/36591. Aerosol formulations, free of propellant gas, comprising a pharmaceutically acceptable salt of tiotropium dissolved in water are disclosed in the international application WO03/084519. Methods for the production of micronized crystalline tiotropium bromide are disclosed in WO03/078429.

For the above-mentioned prophylactic and therapeutic uses the dosages administered will, of course vary with the first and second active compound employed, the treatment desired and the disorder indicated.

The active compounds are dosed in an order of magnitude customary for the individual dose, it more likely being possible, on account of the individual actions, which are mutually positively influencing and reinforcing, to reduce the respective doses on the combined administration of the active compounds compared with the norm.

For inhalation, ipratropium bromide is administered in a dose of preferably 1 to 3 mg per day by once, twice, three or four times daily administration; oxitropium bromide is administered in a dose of preferably 0.2 to 0.6 mg per day by once, twice or three times daily administration; tiotropium bromide monohydrate is administered in a dose of 10 to 25 µg, preferably 22.5 µg per day by once daily administration.

For inhalation, 3-cyclopropylmethoxy-4-difluoromethoxy-N-(3,5-dichloropyrid-4-yl)benzamide (ROFLUMILAST) is administered in a dose of 100 µg to 1000 µg, preferably 250 µg to 500 µg per day, preferably by once daily administration.

For intranasal administration, 3-cyclopropylmethoxy-4-difluoromethoxy-N-(3,5-dichloropyrid-4-yl)benzamide (ROFLUMILAST) is administered in a dose of 100 µg to 1000 µg, preferably 250 µg to 500 µg per day, preferably by once daily administration.

### Preparation Examples:

There follows a description of several Examples showing preparation of pharmaceutical compositions containing a combination of active compounds in accordance with the present invention. These examples are intended to further illustrate the combinations of active compounds of the present invention, pharmaceutical compositions containing them and processes in accordance with which said pharmaceutical compositions may be readily prepared by a person skilled in the art. The person skilled in the art will be aware of many other suitable processes and pharmaceutically acceptable carriers that are also available, as well as acceptable variations in the procedures and ingredients described below.

### Example 1: Dry Powder Inhaler (mono dose system based on capsule for inhalation)

2.50 g micronized ROFLUMILAST, 0.225 g micronized tiotropium bromide monohydrate and 47.3 g lactose monohydrate are mixed in a turbula mixer in two steps. The blend is screened (0.71 mm sieve) to break up any agglomerates and, subsequently, transferred into the container of a planetary mixer. After adding additional 200.0 g lactose monohydrate and mixing, 25 mg of the blend are filled into hard gelatin capsules size #3 using a capsule filling machine. The capsules can be administered with a commercially available inhaler, e.g., the Cyclohaler®. One capsule contains 250 µg of ROFLUMILAST and 22.5 µg of tiotropium bromide monohydrate.

### Example 2: Dry Powder Inhaler (multi dose system)

1.50 g of micronized tiotropium bromide monohydrate and 14.5 g of deagglomerated lactose monohydrate are screened (0.5 mm sieve) and mixed in a turbula mixer until homogenous. The blend obtained is screened (0.5 mm sieve) and filled into a stainless steel container together with 6.67 g of screened ROFLUMILAST (0.5 mm sieve) and 227.4 g of deagglomerated lactose monohydrate and blended in a turbula mixer for a predetermined time. Another 250.0 g of deagglomerated lactose monohydrate are added to the blend. The powders are blended in a turbula mixer until homogenous. 1.2 g of the blend are then filled into the reservoir of a multi dose powder inhaler. After fully assembling, the powder inhaler is pouched into a moisture protective aluminum foil.

Such dry powder inhaler may contain 120 individual doses of 7.5 mg powder each containing 100 µg of ROFLUMILAST and 22.5 µg of tiotropium bromide monohydrate.

### Pharmacology

Inhibition of Methacholine-induced Bronchoconstriction in Guinea Pigs by ROFLUMILAST in combination with tiotropium-bromide

### Objective

To assess the inhibitory effect of tiotropium-bromide, ROFLUMILAST, and the combination of both compounds on methacholine-induced bronchoconstriction in anaesthetized, mechanically ventilated guinea pigs.

### Animals

Male Dunkin Hartley guinea pigs; body weight 350-450 g when performing the experiments.

### Experimental procedure

75 min before methacholine-induced bronchospasm (at -75 min) animals were anaesthetized with urethane i.p. (1.2 g/kg). At -55 min for i.v. injections the right jugular vein and for ventilation the trachea was cannulated. At -45 min NaCl 0.9% or tiotropium-bromide was administered i.v. (1 µg/kg). At -30 min lactose (10 mg/kg) or ROFLUMILAST (4 mg/kg) mixed with lactose was administered intratracheally by a dry powder aerolizer. At -10 min pancuronium-bromide (1.5 mg/kg) was administered i.v. to abolish spontaneous breathing. Animals were mechanically ventilated with 60 breath/min and a tidal volume of 7 ml/kg. Dynamic lung compliance (COM) and airway conductance (CON) were calculated with the help of a computer system from airflow and ventilation pressure signals. At t=0 min methacholine was administered i.v. (60 µg/kg) to induce bronchoconstriction.

### Analysis of lung physiology data

COM and CON were determined up to 120 s after methacholine-induced bronchospasm. AUCs for 0 to 120s were determined. Inhibition was calculated based on the AUC data. Data are shown as mean ± SEM. Results were taken to be significant if p<0.05 versus placebo (ANOVA and Dunnett's multiple comparison test)

### Results

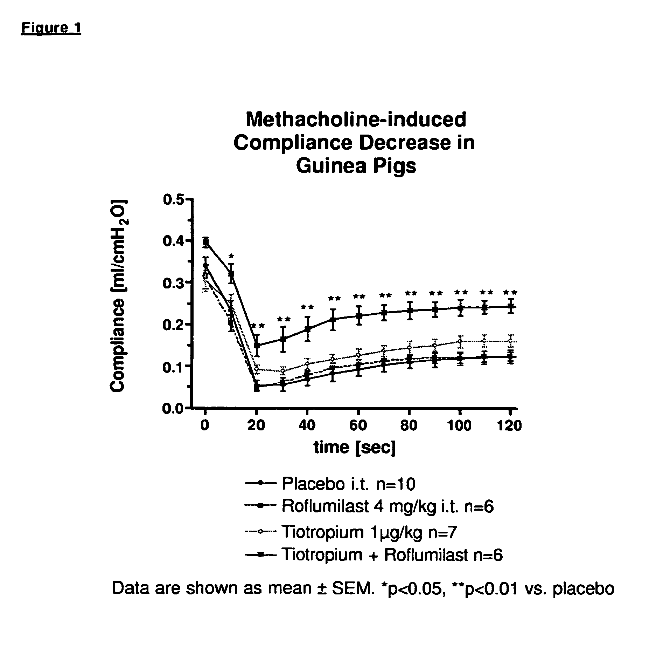

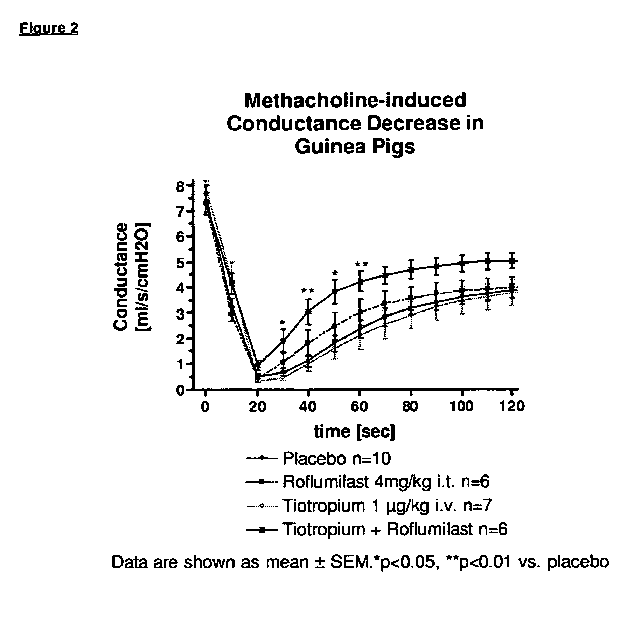

Injection of methacholine induced an immediate bronchoconstriction characterized by a decrease of COM and CON; maximum at 20 s (Fig.1 and Fig. 2).

Pretreatment with ROFLUMILAST had no significant effect on methacholine-induced bronchospasm (Fig. 1 - 4, COM -1.6 %, CON 5.6 %).

Pretreatment with tiotropium-bromide had no significant effect on methacholine-induced bronchospasm (Fig. 1 - 4, COM 12 %, CON 5.9 %).

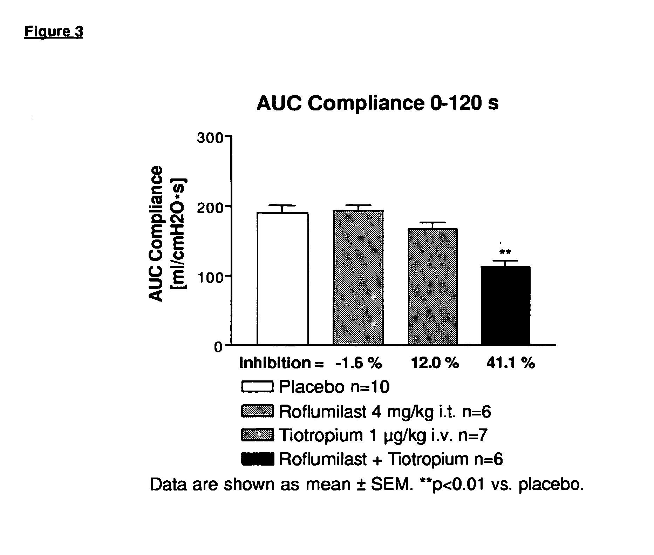

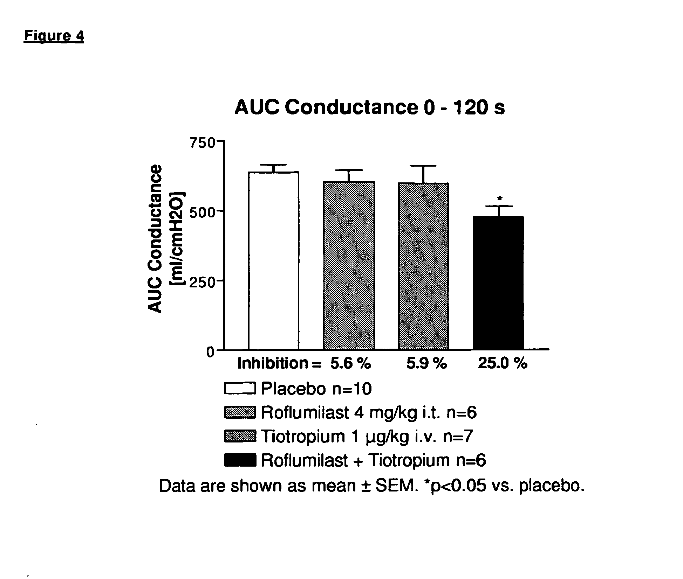

Combination of both treatments led to an unexpected synergistic significant (p<0.01) inhibition of methacholine-induced COM decrease (Fig. 1 and 3, COM 41 %) and CON decrease (Fig. 2 and 4, CON 25 %, p<0.05).

### Conclusion

Whereas ROFLUMILAST and tiotropium-bromide alone had no influence on methacholine-induced bronchospasm in aneasthetized and mechanically ventilated guinea pigs, combination of both active compounds showed an unexpected synergistic inhibition.

### Description of the Figures:

Figure 1: Methacholine induced compliance decrease in guinea pigs
Figure 2: Methacholine induced conductance decrease in guinea pigs
Figure 3: AUC Compliance 0-120 s
Figure 4: AUC Conductance 0-120 s

## Claims

1. Pharmaceutical composition suited for administration by inhalation, which comprises 3-cyclopropylmethoxy-4-difluoromethoxy-N-(3,5-dichloropyrid-4-yl)benzamide, a pharmaceutically acceptable salt thereof or 3-cyclopropylmethoxy-4-difluoromethoxy-N-(3,5-dichloro-1-oxypyrid-4-yl)benzamide and an anticholinergic agent selected from the group of tiotropium bromide and tiotropium bromide monohydrate together with pharmaceutically acceptable excipients and/or carriers in a fixed or free combination.

2. A pharmaceutical composition according to claim 1, which comprises 3-cyclopropylmethoxy-4-difluoromethoxy-N-(3,5-dichloropyrid-4-yl)benzamide and an anticholinergic agent selected from the group of tiotropium bromide or tiotropium bromide monohydrate together with pharmaceutically acceptable excipients and/or carriers in a fixed or free combination.

3. A pharmaceutical composition according to claim 1, which comprises 3-cyclopropylmethoxy-4-difluoromethoxy-N-(3,5-dichloro-1-oxypyrid-4-yl)benzamide and an anticholinergic agent selected from the group of tiotropium bromide or tiotropium bromide monohydrate together with pharmaceutically acceptable excipients and/or carriers in a fixed or free combination.

4. A pharmaceutical composition according to any of claims 1 to 3, wherein the anticholinergic agent is tiotropium bromide.

5. A pharmaceutical composition according to any of claims 1 to 3, wherein the anticholinergic agent is tiotropium bromide monohydrate.

6. Pharmaceutical composition according any of claims 1 to 5, which is a fixed combination.

7. Pharmaceutical composition according to any of claims 1 to 5, which is a free combination.

8. Use of a combination of -3-cyclopropylmethoxy-4-difluoromethoxy-N-(3,5-dichloropyrid-4 yl)benzamide, a pharmaceutically acceptable salt thereof or 3-cyclopropylmethoxy-4-difluoromethoxy-N-(3,5-dichloro-1-oxypyrid-4-yl)benzamide and an anticholinergic agent selected from the group of tiotropium bromide and tiotropium bromide monohydrate for the preparation of a pharmaceutical composition for preventing or reducing the onset of symptoms of a respiratory disease, or treating or reducing the severity of a respiratory disease.

9. Use according to claim 8, wherein the first combination partner is 3-cyclopropylmethoxy-4-difluoromethoxy-N-(3,5-dichloropyrid-4-yl)benzamide.

10. Use according to claim 8, wherein the first combination partner is 3-cyclopropylmethoxy-4-difluoromethoxy-N-(3,5-dichloro-1-oxypyrid-4-yl)benzamide.

11. Use according to any of claims 8 to 10, wherein the anticholinergic agent is tiotropium bromide.

12. Use according to any of claims 8 to 10, wherein the anticholinergic agent is tiotropium bromide monohydrate.

13. Use according to any of claims 8 to 12, wherein the respiratory disease is bronchitis, obstructive bronchitis, spastic bronchitis, allergic bronchitis, allergic asthma, bronchial asthma or COPD.

14. Use according to any of claims 8 to 12, wherein the respiratory disease is COPD.

## Patentansprüche

1. Für die Verabreichung durch Inhalation geeignete pharmazeutische Zusammensetzung, enthaltend 3-Cyclopropylmethoxy-4-difluormethoxy-N-(3,5-dichlorpyrid-4-yl)benzamid, ein pharmazeutisch unbedenkliches Salz davon oder 3-Cyclopropylmethoxy-4-difluormethoxy-N-(3,5-dichlor-1-oxypyrid-4-yl)-benzamid und ein anticholinerges Mittel, ausgewählt aus der Gruppe bestehend aus Tiotropiumbromid und Tiotropiumbromid-monohydrat, zusammen mit pharmazeutisch unbedenklichen Exzipienten und/oder Trägern in fixer oder freier Kombination.

2. Pharmazeutische Zusammensetzung nach Anspruch 1, enthaltend 3-Cyclopropylmethoxy-4-difluormethoxy-N-(3,5-dichlorpyrid-4-yl)benzamid und ein anticholinerges Mittel, ausgewählt aus der Gruppe bestehend aus Tiotropiumbromid und Tiotropiumbromid-monohydrat, zusammen mit pharmazeutisch unbedenklichen Exzipienten und/oder Trägern in fixer oder freier Kombination.

3. Pharmazeutische Zusammensetzung nach Anspruch 1, enthaltend 3-Cyclopropylmethoxy-4-difluormethoxy-N-(3,5-dichlor-1-oxypyrid-4-yl)benzamid und ein anticholinerges Mittel, ausgewählt aus der Gruppe bestehend aus Tiotropiumbromid und Tiotropiumbromid-monohydrat, zusammen mit pharmazeutisch unbedenklichen Exzipienten und/oder Trägern in fixer oder freier Kombination.

4. Pharmazeutische Zusammensetzung nach einem der Ansprüche 1 bis 3, bei der es sich bei dem anticholinergen Mittel um Tiotropiumbromid handelt.

5. Pharmazeutische Zusammensetzung nach einem der Ansprüche 1 bis 3, bei der es sich bei dem anticholinergen Mittel um Tiotropiumbromid-monohydrat handelt.

6. Pharmazeutische Zusammensetzung nach einem der Ansprüche 1 bis 5, bei der es sich um eine fixe Kombination handelt.

7. Pharmazeutische Zusammensetzung nach einem der Ansprüche 1 bis 5, bei der es sich um eine freie Kombination handelt.

8. Verwendung einer Kombination von 3-Cyclopropylmethoxy-4-difluormethoxy-N-(3,5-dichlorpyrid-4-yl)benzamid, einem pharmazeutisch unbedenklichen Salz davon oder 3-Cyclopropylmethoxy-4-difluormethoxy-N-(3,5-dichlor-1-oxypyrid-4-yl)-benzamid und einem anticholinergen Mittel, ausgewählt aus der Gruppe bestehend aus Tiotropiumbromid und Tiotropiumbromid-monohydrat, zur Herstellung einer pharmazeutischen Zusammensetzung zur Prävention oder Verminderung des Einsetzens der Symptome einer Atemwegserkrankung oder zur Behandlung oder Verminderung des Schweregrades einer Atemswegserkrankung.

9. Verwendung nach Anspruch 8, wobei es sich bei dem ersten Kombinationspartner um 3-Cyclopropylmethoxy-4-difluormethoxy-N-(3,5-dichlorpyrid-4-yl)benzamid handelt.

10. Verwendung nach Anspruch 8, wobei es sich bei dem ersten Kombinationspartner um 3-Cyclopropylmethoxy-4-difluormethoxy-N-(3,5-dichlor-1-oxypyrid-4-yl)benzamid handelt.

11. Verwendung nach einem der Ansprüche 8 bis 10, wobei es sich bei dem anticholinergen Mittel um Tiotropiumbromid handelt.

12. Verwendung nach einem der Ansprüche 8 bis 10, wobei es sich bei dem anticholinergen Mittel um Tiotropiumbromid-monohydrat handelt.

13. Verwendung nach einem der Ansprüche 8 bis 12, wobei es sich bei der Atemwegserkrankung um Bronchitis, obstruktive Bronchitis, spastische Bronchitis, allergische Bronchitis, allergisches Asthma, Bronchialasthma oder COPD (Chronic Obstructive Pulmonary Disease, chronisch-obstruktive Atemswegserkrankung) handelt.

14. Verwendung nach einem der Ansprüche 8 bis 12, wobei es sich bei der Atemwegserkrankung um COPD handelt.

## Revendications

1. Composition pharmaceutique appropriée à une administration par inhalation, qui comprend du 3-cyclopropylméthoxy-4-difluorométhoxy-N-(3,5-dichloropyrid-4-yl)-benzamide, un sel pharmaceutiquement acceptable de celui-ci ou du 3-cyclopropylméthoxy-4-difluorométhoxy-N-(3,5-dichloro-1-oxypyrid-4-yl)-benzamide et un agent,anticholinergique choisi dans le groupe constitué du bromure de tiotropium et du bromure de tiotropium monohydraté conjointement avec des excipients et/ou des supports pharmaceutiquement acceptables en combinaison fixe ou libre.

2. Composition pharmaceutique selon la revendication 1, qui comprend du 3-cyclopropylméthoxy-4-difluorométhoxy-N-(3,5-dichloropyrid-4-yl)-benzamide et un agent anticholinergique choisi dans le groupe constitué du bromure de tiotropium et du bromure de tiotropium monohydraté conjointement avec des excipients et/ou des supports pharmaceutiquement acceptables en combinaison fixe ou libre.

3. Composition pharmaceutique selon la revendication 1, qui comprend du 3-cyclopropylméthoxy-4-difluorométhoxy-N-(3,5-dichloro-1-oxypyrid-4-yl)-benzamide et un agent anticholinergique choisi dans le groupe constitué du bromure de tiotropium et du bromure de tiotropium monohydraté conjointement avec des excipients et/ou des supports pharmaceutiquement acceptables en combinaison fixe ou libre.

4. Composition pharmaceutique selon l'une quelconque des revendications 1 à 3, dans laquelle l'agent anticholinergique est le bromure de tiotropium.

5. Composition pharmaceutique selon l'une quelconque des revendications 1 à 3, dans laquelle l'agent anticholinergique est le bromure de tiotropium monohydraté.

6. Composition pharmaceutique selon l'une quelconque des revendications 1 à 5, qui est une combinaison fixe.

7. Composition pharmaceutique selon l'une quelconque des revendications 1 à 5, qui est une combinaison libre.

8. Utilisation d'une combinaison de 3-cyclopropylméthoxy-4-difluorométhoxy-N-(3,5-dichloropyrid-4-yl)-benzamide, d'un sel pharmaceutiquement acceptable de celui-ci ou de 3-cyclopropylméthoxy-4-difluorométhoxy-N-(3,5-dichloro-1-oxypyrid-4-yl)-benzamide et d'un agent anticholinergique choisi dans le groupe constitué du bromure de tiotropium et du bromure de tiotropium monohydraté, pour la préparation d'une composition pharmaceutique destinée à prévenir ou diminuer l'apparition des symptômes d'une maladie respiratoire, ou à traiter ou diminuer la gravité d'une maladie respiratoire.

9. Utilisation selon la revendication 8, selon laquelle le premier partenaire de la combinaison est le 3-cyclopropylméthoxy-4-difluorométhoxy-N-(3,5-dichloropyrid-4-yl)-benzamide.

10. Utilisation selon la revendication 8, selon laquelle le premier partenaire de la combinaison est le 3-cyclopropylméthoxy-4-difluorométhoxy-N-(3,5-dichloro-1-oxypyrid-4-yl)-benzamide.

11. Utilisation selon l'une quelconque des revendications 8 à 10, selon laquelle l'agent anticholinergique est le bromure de tiotropium.

12. Utilisation selon l'une quelconque des revendications 8 à 10, selon laquelle l'agent anticholinergique est le bromure de tiotropium monohydraté.

13. Utilisation selon l'une quelconque des revendications 8 à 12, selon laquelle la maladie respiratoire est la bronchite, la bronchite obstructive, la bronchite spastique, la bronchite allergique, l'asthme allergique, l'asthme bronchique ou la BPCO.

14. Utilisation selon l'une quelconque des revendications 8 à 12, selon laquelle la maladie respiratoire est la BPCO.
